# EUROPÄISCHE PATENTSCHRIFT

(11) **EP 1 648 344 B1**
(45) Veröffentlichungstag und Bekanntmachung des Hinweises auf die Patenterteilung: **16.09.2009**
(21) Anmeldenummer: 04803507.5
(22) Anmeldetag: 03.12.2004
(51) Int. Cl.: A61F 2/06

(54) **MR-KOMPATIBLES MEDIZINISCHES IMPLANTAT**
MAGNETIC RESONANCE-COMPATIBLE MEDICAL IMPLANT
IMPLANT MÉDICAL COMPATIBLE AVEC IMAGERIE PAR RÉSONANCE MAGNÉTIQUE

(30) Priorität: 05.12.2003 DE 10357334
(43) Veröffentlichungstag der Anmeldung: 26.04.2006
(73) Patentinhaber: Grönemeyer, Dietrich H.W., 45549 Sprockhövel (DE)
(72) Erfinder: BUSCH, Martin, 58455 Witten (DE); Grönemeyer, Dietrich H. W., 45549 Sprockhövel (DE)
(74) Vertreter: Isfort, Olaf
(86) Internationale Anmeldenummer: PCT/EP2004/013786
(87) Internationale Veröffentlichungsnummer: WO 2005/053575

(56) Entgegenhaltungen:
- WO-A-02/30331
- WO-A-03/015662
- WO-A-20/05013856
- US-A- 5 667 523
- US-A1- 2002 188 345
- US-B1- 6 231 516
- US-B1- 6 280 385
- FRITZSCHE S, THULL R, HAASE A: "Reduction of NMR image artefacts by using optimal materials as diagnostic aids and implants" BIOMEDIZINISCHE TECHNIK, Bd. 39, Nr. 3, März 1994 (1994-03), Seiten 42-46, XP002024164 Berlin

## Beschreibung

Die Erfindung betrifft ein medizinisches Implantat oder Instrument, insbesondere eine Gefäßendoprothese, mit einem deformierbaren Strukturteil, welches zwei- oder mehrschichtig aufgebaut ist, wobei die Schichten unterschiedliche elektrische und/oder magnetische Eigenschaften haben, wobei das Strukturteil eine durch eine Mehrzahl von miteinander verbundenen, metallischen Streben gebildete, ausdehnbare Gerüststruktur aufweist, die derart ausgebildet ist, dass in sich geschlossene Strompfade innerhalb einzelner Schichten des Strukturteils vermieden werden, und wobei die Gerüststruktur Unterbrechungen aufweist, die sich in unterschiedlichen Schichten des Strukturteils jeweils an unterschiedlichen, nicht direkt übereinander liegenden Positionen befinden.

Gefäßendoprothesen (so genannte "Stents") und andere medizinische Implantate oder Instrumente, die ein deformierbares Strukturteil aufweisen, wie z. B. intravaskuläre Filtersysteme, sind aus dem Stand der Technik bekannt. Das Strukturteil wird bei Stents üblicherweise durch gitterförmig angeordnete Metallfilamente gebildet, die zur Abstützung und/oder zur Glättung einer geschädigten Koronargefäßwand eingesetzt werden. Ein Stent wird - ähnlich wie bei einer PTCA-Behandlung - mittels eines Ballonkatheters im Bereich der geschädigten Stelle des zu behandelnden Gefäßes positioniert. Stents werden hauptsächlich eingesetzt, um akute Gefäßverschlüsse oder Restenosierungen nach PTCA-Behandlungen zu verhindern. So genannte Stent-Grafts werden zur Behandlung von Aneurysmen eingesetzt.

Die diagnostische Bildgebung von Bereichen in der Umgebung eines Stents oder eines ähnlichen medizinischen Implantats mittels magnetischer Resonanz (MR) erweist sich oft als problematisch. Dies kann zum einen darauf zurückzuführen sein, dass das Strukturteil des im Körper des untersuchten Patienten befindlichen Implantats aus paramagnetischem Material besteht. Durch die magnetische Suszeptibilität des Implantats wird das Magnetfeld in der ansonsten diamagnetischen Umgebung des Implantats verzerrt, so dass in den aufgenommenen Bildern Artefakte entstehen. Diese artefaktbehafteten Bilder sind dann für diagnostische Zwecke zumeist nicht brauchbar. Medizinische Implantate und Instrumente, die ein aus metallischen Filamenten bestehendes Strukturteil aufweisen, haben zudem den Nachteil, dass die gitter- oder netzartige Struktur bei der MR-Bildgebung als Faraday-Käfig wirkt, so dass die bei der MR-Bildgebung eingestrahlten Hochfrequenzfelder nicht in das Volumen innerhalb des Implantates eindringen. Aufgrund dieser Abschirmung bleibt das Innere eines herkömmlichen Stents bei der MR-Bildgebung nachteiligerweise oft unsichtbar. Nachteilig ist dies insbesondere, weil verhindert wird, dass mittels MR-Bildgebung eine Restenosierung im Inneren eines Stents frühzeitig diagnostiziert werden kann.

Aus der US 2002/0188345 A1 ist ein MR-kompatibler Stent bekannt, dessen deformierbares Strukturteil aus Metallfilamenten besteht, die an bestimmten Stellen Unterbrechungen aufweisen, so dass in sich geschlossene Strompfade innerhalb der Gerüststruktur unterbunden werden. Durch die Verhinderung von Wirbelströmen wird damit erreicht, dass der Stent nicht als Faraday-Käfig wirken kann. Die bei der MR-Bildgebung eingestrahlten Hochfrequenzfelder können also keine Wirbelströme in der Gerüststruktur des Stents induzieren, und eine MR-Bildgebung auch des Inneren des Stents wird ermöglicht. Nachteilig ist bei dem aus der genannten US-Offenlegungsschrift vorbekannten Stent allerdings, dass dessen Herstellung extrem aufwendig und teuer ist. Damit die strukturelle Integrität des Stents nicht leidet, müssen nämlich die zur Unterbindung von Wirbelströmen angebrachten Unterbrechungen mit geeignetem elektrisch nicht leitfähigem Material überbrückt werden. Hierzu kommen Klebstoffe, Kunststoffe oder Ähnliches in Frage. Damit die mit Unterbrechungen versehenen Metallfilamente außerdem eine ausreichende Stabilität bei Scherbelastungen aufweisen, müssen die Unterbrechungen bei dem vorbekannten Stent zusätzlich eine spezielle Formgebung aufweisen, die in der genannten US 2002/0188345 A1 im Einzelnen beschrieben ist. Die Herstellung dieser speziell geformten Unterbrechungen ist besonders aufwendig und technologisch schwierig. Nachteilig ist bei dem vorbekannten Stent ferner, dass keinerlei Vorkehrungen getroffen sind, um die oben erwähnten Suszeptibilitätsartefakte in den mittels MR-aufgenommenen Bildern zu minimieren.

Eine MR-kompatible metallische Endoprothese der eingangs genannten Art ist aus der WO 03/015662 A1 vorbekannt. Bei der vorbekannten Endoprothese wird durch Kombination des Herstellungsmaterials mit einem speziellen Design der Gerüststruktur des Strukturteils erzielt, dass die Endoprothese in MR-Bildern keinerlei Artefakte hervorruft und ggf. auch eine Bildgebung des Lumens der Endoprothese mittels MR möglich ist. Das Design der vorbekannten Endoprothese ist derart, dass die Streben der Gerüststruktur weitgehend entlang der Längsachse der Endoprothese verlaufen, um keine geschlossenen Strompfade in einer Ebene im Wesentlichen senkrecht zur Endoprothesenlängsachse zu bilden. Die vorbekannte Prothese kann einen mehrschichtigen Aufbau haben. Offenbart ist beispielsweise eine Ummantelung mit einer oder mehreren Membranen aus Polymermaterial. Nachteilig ist bei der vorbekannten Endoprothese wiederum vor allem deren vergleichsweise aufwendige Herstellung.

Die US 6,231,516 B1 betrifft ein endoluminales Implantat, das mit einer Hochfrequenzspule verbunden ist. Über die Hochfrequenzspule kann dem Implantat elektrische Energie zu therapeutischen Zwecken zugeführt werden. Das vorbekannten Implantat kann einen mehrschichtigen Aufbau haben, wobei die Hochfrequenzspule helixförmig um den Umfang eines Stents herumgelegt ist. MR-kompatibel ist das vorbekannte Implantat nachteiligerweise nicht.

Ein MR-kompatibler Stent ist des Weiteren aus der US 6,280,385 B1 bekannt. Dieser Stent weist einen passiven Resonanzkreis mit einer Induktivität und einer Kapazität auf, wobei die Resonanzfrequenz auf die Frequenz des verwendeten MR-Systems abgestimmt ist. Das Strukturteil des vorbekannten Stents kann einen mehrschichtigen Aufbau haben, wobei die verschiedenen Schichten unterschiedliche elektrische Leitfähigkeiten haben. Durch Strukturierung der Schichten wird die für die Ausbildung des Resonanzkreises erforderliche Induktivität erzeugt.

In dem Artikel "Reduzierung von NMR-Bildartefakte durch Benutzung optimierter Werkstoffe für diagnostische Hilfsmittel und Implantate" von S. Fritzsche, R. Thull und A. Haase in der Zeitschrift Biomedizinische Technik, Band 36, Heft 3/1994, Seiten 42 bis 46, wird für medizinische Implantate die Verwendung von Werkstoffen angesprochen, die in ihrer magnetischen Suszeptibilität an die jeweilige Umgebung angepasst sind. Hierdurch soll erzielt werden, dass Artefakte und Signalverlust bei der MR-Bildgebung von Bereichen in der Umgebung eines Implantats im menschlichen Körper vermieden werden.

Eine weitere Gefäßendoprothese ist aus der US 5,667,523 vorbekannt. Diese Druckschrift betrifft einen Stent mit einem radial aufweitbaren Strukturteil, das einen mehrschichtigen Aufbau aufweist. MR-kompatibel ist der vorbekannte Stent nachteiligerweise nicht.

Die WO 2005/013856 A2, ein Dokument, welches unter Artikel 54(3) EPÜ fällt, betrifft eine mehrschichtige Ausgestaltung eines MR-kompatiblen Stents, wobei zwei elektrisch leitende Schichten vorgesehen sind, die jeweils zur Unterbindung der ungewünschten Ausbildung von geschlossenen Stromkreisen mit Unterbrechungen versehen sind, die in den unterschiedlichen Schichten jeweils an unterschiedlichen, nicht übereinanderliegenden Positionen angeordnet sind, wodurch insgesamt ein Stent hoher struktureller Integrität bereitgestellt wird, welcher im Vergleich zu den vorbeschriebenen Ausgestaltungen von Endoprothesen kostengünstig herstellbar ist.

Davon ausgehend liegt der vorliegenden Erfindung die Aufgabe zugrunde, ein MR-kompatibles medizinisches Implantat oder Instrument, insbesondere einen Stent, bereitzustellen, mit dem die MR-Bildgebung des Volumens innerhalb eines gemäß der Erfindung ausgebildeten medizinischen Implantats oder Instrumentes besonders gut möglich ist.

Diese Aufgabe löst die Erfindung ausgehend von einem medizinischen Implantat oder Instrument der eingangs genannten Art dadurch, dass die Unterbrechungen derart angeordnet sind, dass innerhalb wenigstens einer Schicht ein sich von einem Endbereich des Strukturteils zum gegenüberliegenden Endbereich hin erstreckender durchgehender Strompfad gebildet wird.

Die MR-Kompatibilität des erfindungsgemäßen medizinischen Implantates resultiert zum einen aus dessen mehrschichtigem Aufbau. Der mehrschichtige Aufbau hat vor allem den Vorteil einer besonders einfachen und kostengünstigen Herstellbarkeit des erfindungsgemäßen medizinischen Implantates. Das deformierbare Strukturteil eines gemäß der Erfindung aufgebauten Stents kann beispielsweise aus zwei Rohrteilen mit unterschiedlichen Durchmessern bestehen, die koaxial ineinander angeordnet sind. Dabei kann jedes einzelne Rohrteil, wie bei herkömmlichen Stents, beispielsweise mittels eines Lasers durch Herausschneiden einer Gerüststruktur aus einem durchgehenden Rohr derart strukturiert werden, dass die beiden Rohrteile die gewünschten unterschiedlichen elektrischen Eigenschaften haben. Nach oder Strukturierung der Rohrteile können diese ineinander angeordnet und unter Verwendung eines geeigneten Klebers miteinander verklebt werden. Genauso gut können die Rohrteile zuerst ineinander angeordnet und miteinander verklebt werden und erst danach mittels eines Lasers oder sonst wie in der erfindungsgemäßen Weise strukturiert werden. Unterschiedliche magnetische Eigenschaften ergeben sich durch geeignete Wahl der Materialien für die beiden Rohrteile.

Ähnlich wie bei herkömmlichen Stents weist das Strukturteil des erfindungsgemäßen medizinischen Implantats bzw. Instrumentes eine durch eine Mehrzahl von miteinander verbundenen metallischen Streben gebildete, ausdehnbare Gerüststruktur auf. Damit die gewünschte MR-Kompatibilität gewährleistet ist, weist die Gerüststruktur, beispielsweise durch Durchtrennung einzelner Streben, Unterbrechungen auf, derart, dass in sich geschlossene Strompfade innerhalb einzelner Schichten des Strukturteils vermieden werden. Dadurch werden bei der MR-Bildgebung auftretende Wirbelströme wirksam verhindert, so dass das erfindungsgemäße medizinische Implantat die eingestrahlten Hochfrequenzfelder nicht abschirmt. Wesentlich ist, dass sich die Unterbrechungen in unterschiedlichen Schichten des Strukturteils jeweils an unterschiedlichen, nicht direkt übereinanderliegenden Positionen befinden. Dadurch dass an einer Position, an der sich in einer Schicht eine Unterbrechung befindet, in einer anderen Schicht keine Unterbrechung vorgesehen ist, ist die strukturelle Integrität der erfindungsgemäßen Gesamtanordnung gewährleistet, was insbesondere bei Stents wichtig ist, damit diese den von den Gefäßwandungen aufgebrachten radialen Belastungen standhalten können. Zur Sicherstellung einer ausreichenden strukturellen Integrität ist gemäß der Erfindung ein deutlich geringerer Herstellungsaufwand nötig als dies bei vorbekannten Stent-Strukturen der Fall ist.

Erfindungsgemäß sind die Unterbrechungen derart angeordnet sind, dass innerhalb wenigstens einer Schicht ein sich von einem Endbereich des Strukturteils zum gegenüberliegenden Endbereich hin erstreckender durchgehender Strompfad gebildet wird. Dieser durchgehende Strompfad kann vorteilhafterweise helixförmig ausgebildet sein, so dass die einzelnen Schichten jeweils die elektrischen Eigenschaften einer Induktivität haben. Sinnvoll ist es dabei, wenn die innerhalb unterschiedlicher Schichten des Strukturteils gebildeten durchgehenden Strompfade miteinander verbunden sind, so dass sich die Induktivitäten der einzelnen Schichten je nach Bedarf in geeigneter Weise zu einer Gesamtimpedanz addieren. Besonders vorteilhaft ist es, die durchgehenden Strompfade der unterschiedlichen Schichten über wenigstens eine elektrische Kapazität miteinander zu verbinden. So entsteht insgesamt eine Resonatorstruktur, die bei der MR-Bildgebung genutzt werden kann. Hierzu muss die Resonanzfrequenz der Struktur auf die Resonanzfrequenz des MR-Gerätes abgestimmt sein. Die bei der MR-Bildgebung eingestrahlten Hochfrequenzfelder werden dann nicht, wie bei herkömmlichen Stents, abgeschirmt, sondern - im Gegenteil - im Inneren des Implantats sogar verstärkt. Damit ist dann die MR-Bildgebung des Volumens innerhalb eines gemäß der Erfindung ausgebildeten Stents besonders gut möglich. Die für die Wirkung als Resonator erforderliche Kapazität kann besonders einfach durch übereinander liegende, elektrisch leitende Bereiche der Schichten des Strukturteils des erfindungsgemäßen medizinischen Implantates gebildet werden. Je nach Anwendungsfall können die innerhalb der einzelnen Schichten gebildeten durchgehenden Strompfade auch über Durchkontaktierungen in den Endbereichen des Strukturteils miteinander verbunden sein. Durch derartige Verbindungen kann gezielt die gewünschte Gesamtimpedanz des medizinischen Implantates eingestellt werden. Wenn eine möglichst große Induktivität angestrebt wird, ist es zweckmäßig, wenn die helixförmig ausgebildeten Strompfade unterschiedlicher Schichten entgegengesetzten Drehsinn haben, derart, dass sich die Induktivitäten der miteinander verbundenen einzelnen Schichten addieren und nicht etwa kompensieren.

Eine alternative Möglichkeit, durch das erfindungsgemäße Implantat bzw. Instrument eine Resonatorstruktur zu erzeugen, besteht darin, die Unterbrechungen in der Gerüststruktur derart anzuordnen, dass innerhalb wenigstens zweier übereinander liegender Schichten des Strukturteils zwei oder mehr im Wesentlichen helixförmige Strompfadabschnitte gebildet werden, wobei die Strompfadabschnitte unterschiedlicher Schichten des Strukturteils einander zumindest teilweise überdeckend angeordnet sind. Demnach weist jede einzelne Schicht zwei oder mehr hintereinander geschaltete helixförmige Strompfadabschnitte auf, die jeweils mit ihrer Induktivität zu der Gesamtinduktivität des Resonators beitragen. Die helixförmigen Strompfadabschnitte unterschiedlicher Schichten überdecken sich dabei teilweise, so dass in den Überdeckungsbereichen Kapazitäten gebildet werden. Insgesamt ist diese Struktur nach Art eines auf dem Gebiet der Hochfrequenztechnik bekannten, so genannten Split-Ring-Resonators aufgebaut. Dabei bewirkt die Hintereinanderanordnung von zwei oder mehr helixförmigen Strompfadabschnitten, die jeweils mehrere Helixwindungen aufweisen können, dass die Gesamtinduktivität der Struktur ausreichend groß ist, damit die Resonanzfrequenz im Bereich üblicher MR-Resonanzfrequenzen von ca. 20 bis 400 MHz liegt.

Um die angesprochenen magnetischen Suszeptibilitätsartefakte bei Verwendung des erfindungsgemäßen medizinischen Implantates bei der MR-Bildgebung zu vermeiden, ist es besonders zweckmäßig, dass wenigstens zwei der Schichten des Strukturteils aus Materialien mit zueinander entgegengesetzten magnetischen Suszeptibilitäten bestehen. Demnach sollte wenigstens eine der Schichten aus einem diamagnetischen Material bestehen, während wenigstens eine weitere Schicht aus einem ferro- oder paramagnetischen Material besteht. Die entgegengesetzten Suszeptibilitäten kompensieren sich gegenseitig weitgehend, so dass die effektive Gesamtsuszeptibilität des Implantats bzw. des Instruments verringert wird. Es kommt dann nur noch in reduziertem Maße zu Verzerrungen der Magnetfelder in der Umgebung des Implantats, und entsprechende Artefakte in den aufgenommenen MR-Bildern treten vermindert auf.

Es ist zweckmäßig, dass bei dem medizinischen Implantat gemäß der Erfindung die aus elektrisch leitendem Material bestehenden Schichten des Strukturteils durch aus elektrisch isolierendem Material bestehende Schichten voneinander getrennt sind. Dadurch wird vor allem sichergestellt, dass die gewünschten unterschiedlichen elektrischen Eigenschaften der einzelnen Schichten gezielt vorgegeben werden können. Insbesondere, wenn zur Unterbindung der beschriebenen Wirkung des Strukturteils des Implantats als Faraday-Käfig gezielt Unterbrechungen in der Gerüststruktur angebracht werden, müssen elektrische Verbindungen zwischen den verschiedenen Schichten verhindert werden, da ansonsten die Unterbrechungen überbrückt und damit wirkungslos werden könnten.

Das erfindungsgemäße medizinische Implantat kann mit Vorteil bei einem MR-Bildgebungsverfahren zur Erzeugung eines Bildes eines im Untersuchungsvolumen eines MR-Gerätes befindlichen Patienten verwendet werden. Zur Unterdrückung von magnetischen Suszeptibilitätsartefakten kann dem Patienten während der Bildaufnahme ein paramagnetisches Kontrastmittel intravenös appliziert werden, das derart zusammengesetzt ist, dass die paramagnetische Suszeptibilität des Blutes in der Umgebung des medizinischen Implantates im Wesentlichen gleich der paramagnetischen Suszeptibilität des medizinischen Implantates selbst ist. Wenn demnach die Umgebung des medizinischen Implantates die im wesentlichen selben Suszeptibilitätseigenschaften hat wie das medizinischen Implantat selbst, kommt es nicht zu lokalen Verzerrungen der magnetischen Felder. Dementsprechend treten auch keine Artefakte in den aufgenommenen MR-Bildern auf. Geeignete paramagnetische Kontrastmittel können wenigstens einen Stoff aus der Gruppe der Ferrite enthalten. Das heute allgemein übliche Kontrastmittel GdDTPA kann ebenfalls eingesetzt werden.

Ausführungsbeispiele der Erfindung werden im Folgenden anhand der Figuren erläutert. Es zeigen:
- Fig. 1: erfindungsgemäße Gefäßendoprothese im Querschnitt;
- Fig. 2: in die Zeichnungsebene abgewickelte Darstellung der unterschiedlichen Strukturierungen der Schichten des Implantats gemäß Fig. 1;
- Fig. 3: dreidimensionale Ansicht der Koronarendoprothese gemäß Fig. 1;
- Fig. 4: Ersatzschaltbild zur Verdeutlichung der Resonatoreigenschaften des Implantats;
- Fig. 5: schematische Darstellung des erfindungsgemäßen erweiterten Split-Ring- Resonators.

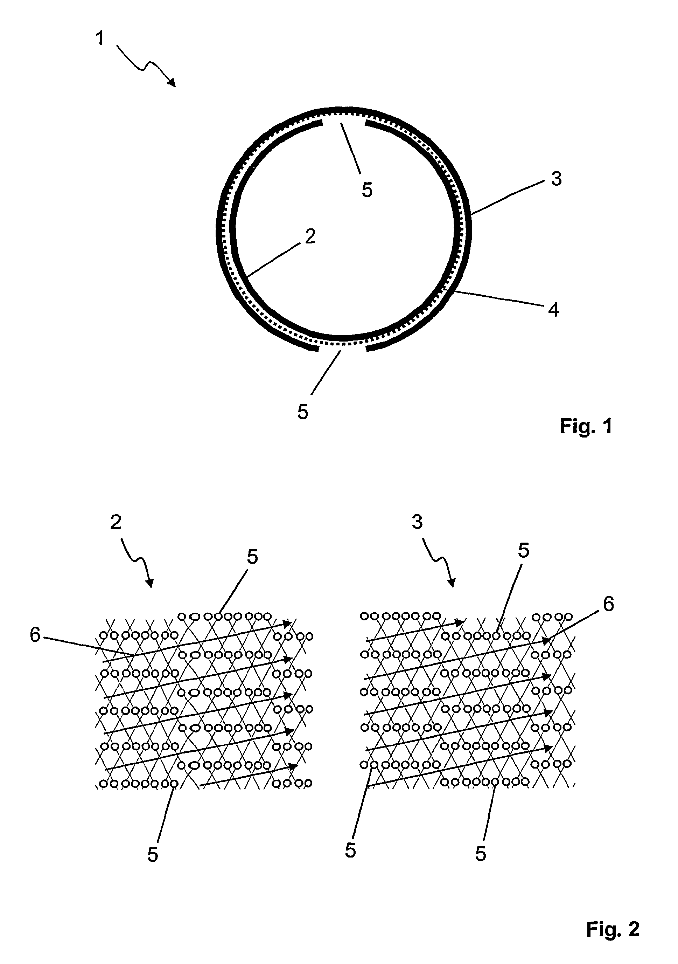

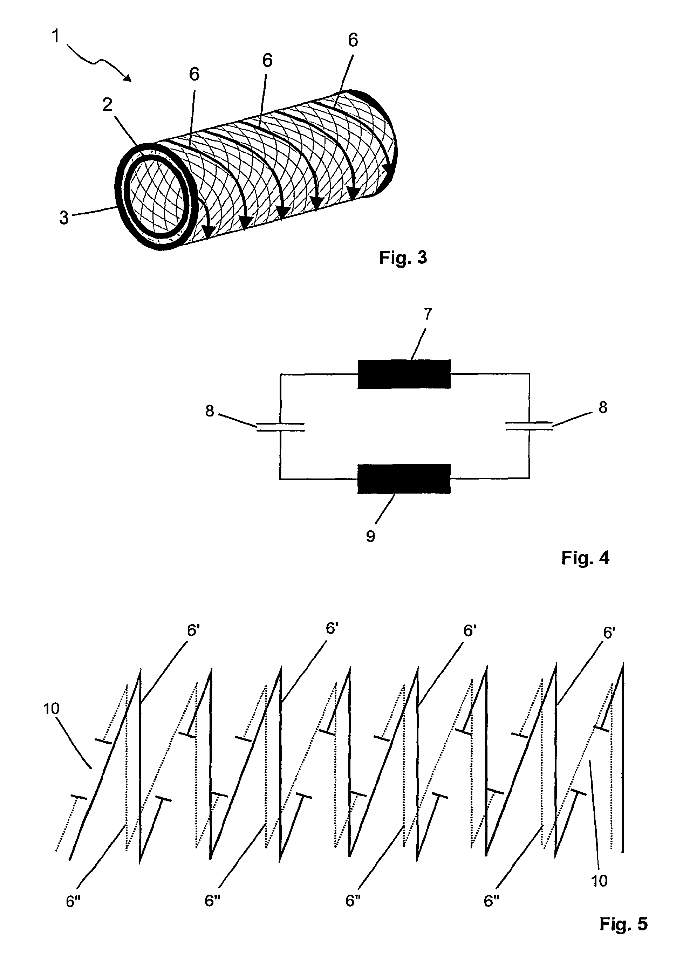

Bei dem in den Figuren dargestellten medizinischen Implantat handelt es sich um einen Stent, der in den Fig. 1 und 3 als Ganzes mit der Bezugsziffer 1 bezeichnet ist. Der Stent 1 weist ein deformierbares, ausdehnbares Strukturteil mit einem zweischichtigen Aufbau aus. Eine innere Schicht 2 und eine äußere Schicht 3 sind übereinander liegend angeordnet. Die Schichten 2 und 3 des Strukturteils werden durch zwei koaxial angeordnete röhrenförmige Elemente gebildet, die in der Fig. 3 gut zu erkennen sind. Die aus elektrisch leitendem Material bestehenden Schichten 2 und 3 sind durch eine aus elektrisch isolierendem Material bestehende Zwischenschicht 4 voneinander getrennt. Dabei kann es sich beispielsweise um eine Schicht aus Klebstoff handeln, mittels welchem die ineinander koaxial angeordneten röhrenförmigen Elemente miteinander verbunden sind.

Bei dem in den Figuren dargestellten medizinischen Implantat weisen die Schichten 2 und 3, die das Strukturteil des Stents bilden, eine durch eine Mehrzahl von miteinander verbundenen metallischen Streben gebildete, ausdehnbare Gerüststruktur auf. Diese Struktur ist in den Fig. 2 und 3 zu erkennen. Die Streben sind Metallfilamente, die bei dem Ausführungsbeispiel insgesamt ein rautenförmiges Gitter bilden. Die Gerüststruktur weist Unterbrechungen 5 auf, die in der Fig. 2 durch offene Kreise symbolisiert sind. Die Fig. 2 zeigt lediglich einen Ausschnitt aus der Gesamtstruktur des Stents. Durch die Unterbrechungen 5 werden in sich geschlossene Strompfade innerhalb der Schichten 2 und 3 des Strukturteils vermieden. Anhand der in die Zeichnungsebene abgewickelten Darstellung gemäß Fig. 2 ist deutlich zu erkennen, dass sich in den Schichten 2 und 3 die Unterbrechungen 5 jeweils an unterschiedlichen, nicht direkt übereinander liegenden Positionen befinden. Dadurch wird, wie oben ausgeführt, sichergestellt, dass die strukturelle Integrität des Stents 1 insgesamt gewährleistet ist. Des Weiteren sind die Unterbrechungen 5 derart angeordnet, dass die röhrenförmigen Schichten 2 und 3 jeweils helixförmige durchgehende Strompfade aufweisen, was in den Fig. 2 und 3 durch Pfeile 6 symbolisiert ist. Die helixförmigen Strompfade 6 bilden Induktivitäten, die über elektrische Kapazitäten miteinander verbunden sind. Diese Kapazitäten werden durch die übereinander liegenden, elektrisch leitenden Bereiche der Schichten 2, 3 des Strukturteils gebildet.

Anhand des Schaltbildes gemäß Fig. 4 wird deutlich, wie die Induktivitäten und Kapazitäten miteinander verschaltet sind. Eine Induktivität 7 ist der inneren Schicht 2 des Stents 1 zugeordnet. Durch die koaxiale Anordnung der röhrenförmigen Elemente des Stents 1 entstehen Kapazitäten 8, über welche die Induktivität 7 der inneren Schicht 2 mit einer Induktivität 9 der äußeren Schicht 3 verbunden ist. Insgesamt entsteht so ein Resonanzkreis, wobei die Kapazität 8 und die durch die Strompfade 6 gebildeten Induktivitäten 7 und 9 derart aufeinander abgestimmt sind, dass die Resonanzfrequenz gleich der Resonanzfrequenz eines in den Figuren nicht näher dargestellten MR-Gerätes ist.

Die Fig. 5 zeigt schematisch die oben beschriebene Split-Ring-Struktur, die sich gemäß der Erfindung durch geeignete Anordnung der Unterbrechungen innerhalb der Gerüststruktur des Implantats erzielen lässt. In der Darstellung gemäß Fig. 5 symbolisieren die durchgezogenen Linien Strompfandabschnitte 6' innerhalb der äußeren Schicht 3 des Resonators, während die gestrichelten Linien Strompfandabschnitte 6" darstellen, die durch geeignete Anordnung der Unterbrechungen innerhalb der Schicht 2 gebildet werden. Die Strompfadabschnitte 6' und 6" sind jeweils helixförmig ausgebildet und umfassen jeweils zwei vollständige Helixwindungen. In der Darstellung der Fig. 5 symbolisieren vertikal ausgerichtete Linien in der Draufsicht auf die gesamte Struktur hinten liegende Abschnitte der Helixwindungen, während die schrägen Linien die vorne liegenden Helixabschnitte darstellen. In der Fig. 5 ist zu erkennen, dass in den Schichten 2 und 3 jeweils vier Strompfadabschnitte 6' bzw. 6" hintereinander angeordnet sind, wobei die einzelnen Strompfadabschnitte jeweils durch isolierende Abschnitte 10 voneinander getrennt sind. Die Anordnung ist derart, dass sich ein isolierender Abschnitt 10 der inneren Schicht 2 etwa im mittleren Bereich eines Strompfadabschnitts 6' der äußeren Schicht 3 befindet. Dadurch ergibt sich insgesamt die einander zumindest teilweise überdeckende Anordnung der Strompfadabschnitte. So entsteht eine erweitere Split-Ring-Struktur, die als MR-Resonator in der oben beschriebenen Weise nutzbar ist.

## Patentansprüche

1. Medizinisches Implantat oder Instrument, insbesondere Gefäßendoprothese (1) mit einem deformierbaren Strukturteil, welches zwei- oder mehrschichtig aufgebaut ist, wobei die Schichten (2, 3) unterschiedliche elektrische und/oder magnetische Eigenschaften haben, wobei das Strukturteil eine durch eine Mehrzahl von miteinander verbundenen, metallischen Streben gebildete, ausdehnbare Gerüststruktur aufweist, die derart ausgebildet ist, dass in sich geschlossene Strompfade innerhalb einzelner Schichten (2, 3) des Strukturteils vermieden werden, und wobei die Gerüststruktur Unterbrechungen aufweist, die sich in unterschiedlichen Schichten (2, 3) des Strukturteils jeweils an unterschiedlichen, nicht direkt übereinander liegenden Positionen befinden, wobei die Unterbrechungen (5) derart angeordnet sind, dass innerhalb wenigstens einer Schicht (2, 3) ein sich von einem Endbereich des Strukturteils zum gegenüberliegenden Endbereich hin erstreckender durchgehender Strompfad (6) gebildet wird.

2. Medizinisches Implantat oder Instrument nach Anspruch 1, **dadurch gekennzeichnet, dass** der durchgehende Strompfad (6) helixförmig ausgebildet ist.

3. Medizinisches Implantat oder Instrument nach Anspruch 1, **dadurch gekennzeichnet, dass** die Unterbrechungen (5) derart angeordnet sind, dass innerhalb wenigstens zweier übereinander liegender Schichten (2, 3) zwei oder mehr im Wesentlichen helixförmige Strompfadabschnitte (6', 6") gebildet werden, wobei die Strompfadabschnitte (6', 6") unterschiedlicher Schichten (2, 3) des Strukturteils einander zumindest teilweise überdeckend angeordnet sind.

4. Medizinisches Implantat oder Instrument nach einem der Ansprüche 1 bis 3, **dadurch gekennzeichnet, dass** die innerhalb unterschiedlicher Schichten (2, 3) des Strukturteils gebildeten durchgehenden Strompfade (6) oder Strompfadabschnitte (6', 6") miteinander verbunden sind.

5. Medizinisches Implantat oder Instrument nach Anspruch 4, **dadurch gekennzeichnet, dass** die Strompfade (6) oder Strompfadabschnitte (6, 6") über wenigstens eine elektrische Kapazität (8) miteinander verbunden sind.

6. Medizinisches Implantat oder Instrument nach Anspruch 5, **dadurch gekennzeichnet, dass** die Kapazität (8) durch übereinander liegende, elektrisch leitende Bereiche der Schichten (2, 3) des Strukturteils gebildet wird.

7. Medizinisches Implantat oder Instrument nach einem der Ansprüche 1 bis 7, **dadurch gekennzeichnet, dass** die Strompfade (6) oder Strompfadabschnitte (6', 6") über Durchkontaktierungen zwischen den Schichten (2, 3) miteinander verbunden sind.

8. Medizinisches Implantat oder Instrument nach Anspruch 2 oder 3, **dadurch gekennzeichnet, dass** die helixförmigen Strompfade (6) oder Strompfadabschnitte (6', 6") unterschiedlicher Schichten (2, 3) entgegengesetzten Drehsinn haben.

9. Medizinisches Implantat oder Instrument nach Anspruch 5, **dadurch gekennzeichnet, dass** die Kapazität (8) und die durch die Strompfade (6) oder Strompfadabschnitte (6', 6") gebildeten Induktivitäten (7, 9) derart aufeinander abgestimmt sind, dass ein Hochfrequenzresonator gebildet wird, dessen Resonanzfrequenz gleich der Resonanzfrequenz eines MR-Gerätes ist.

10. Medizinisches Implantat oder Instrument nach einem der Ansprüche 1 bis 9, **dadurch gekennzeichnet, dass** wenigstens zwei der Schichten (2, 3) des Strukturteils aus Materialien mit zueinander entgegengesetzten magnetischen Suszeptibilitäten bestehen.

11. Medizinisches Implantat oder Instrument nach einem der Ansprüche 1 bis 10, **dadurch gekennzeichnet, dass** die Schichten (2, 3) des Strukturteils durch zwei oder mehr koaxial angeordnete röhrenförmige Elemente gebildet werden.

12. Medizinisches Implantat oder Instrument nach einem der Ansprüche 1 bis 11, **dadurch gekennzeichnet, dass** aus elektrisch leitendem Material bestehende Schichten (2, 3) des Strukturteils durch aus elektrisch isolierendem Material bestehende Zwischenschichten (4) voneinander getrennt sind.

## Claims

1. A medical implant or instrument, in particular a vascular endoprosthesis (1), comprising a deformable structural part which is of a two-layer or multi-layer structure, wherein the layers (2, 3) have different electrical and/or magnetic properties, wherein the structural part has an expandable frame structure which is formed by a plurality of interconnected metallic struts and which is so designed that current paths which are closed in themselves within individual layers (2, 3) of the structural part are avoided, and wherein the frame structure has interruptions disposed in different layers (2, 3) of the structural part at respective different positions which are not in directly mutually superposed relationship, wherein the interruptions (5) are so arranged that within at least one layer (2, 3) there is formed a continuous current path (6) extending from an end region of the structural part to the opposite end region.

2. A medical implant or instrument according to claim 1 **characterised in that** the continuous current path (6) is of a helical configuration.

3. A medical implant or instrument according to claim 1 **characterised in that** the interruptions (5) are so arranged that within at least two mutually superposed layers (2, 3) two or more substantially helical current path portions (6', 6") are formed, wherein the current path portions (6', 6") of different layers (2, 3) of the structural part are arranged in at least partially mutually overlapping relationship.

4. A medical implant or instrument according to one of claims 1 to 3 **characterised in that** the continuous current paths (6) or current path portions (6', 6") formed within different layers (2, 3) of the structural part are connected together.

5. A medical implant or instrument according to claim 4 **characterised in that** the current paths (6) or current path portions (6', 6") are connected together by way of at least one electrical capacitor (8).

6. A medical implant or instrument according to claim 5 **characterised in that** the capacitor (8) is formed by mutually superposed, electrically conducting regions of the layers (2, 3) of the structural part.

7. A medical implant or instrument according to one of claims 1 to 7 **characterised in that** the current paths (6) or current path portions (6', 6") are connected together by way of through-contacting means between the layers (2, 3).

8. A medical implant or instrument according to claim 2 or claim 3 **characterised in that** the helical current paths (6) or current path portions (6', 6") of different layers (2, 3) are of opposite directions of rotation.

9. A medical implant or instrument according to claim 5 **characterised in that** the capacitor (8) and the inductances (7, 9) formed by the current paths (6) or current path portions (6', 6") are matched to each other in such a way that a high frequency generator is formed, the resonance frequency of which is equal to the resonance frequency of an MR apparatus.

10. A medical implant or instrument according to one of claims 1 to 9 **characterised in that** at least two of the layers (2, 3) of the structural part comprise metals of mutually opposite magnetic susceptibilities.

11. A medical implant or instrument according to one of claims 1 to 10 **characterised in that** the layers (2, 3) of the structural part are formed by two or more coaxially arranged tubular elements.

12. A medical implant or instrument according to one or more of claims 1 to 11 **characterised in that** layers (2, 3) of the structural part, that comprise electrically conducting material, are separated from each other by intermediate layers (4) comprising electrically insulating material.

## Revendications

1. Implant ou instrument médical, en particulier endoprothèse vasculaire (1) avec une partie structurelle déformable qui est réalisée en deux ou plusieurs couches, où les couches (2, 3) ont des propriétés électriques et/ou magnétiques différentes, où la partie structurelle présente une structure de charpente extensible, formée par plusieurs montants métalliques reliés les uns aux autres, qui est réalisée de façon à éviter des trajets de courant fermés en soi à l'intérieur des différentes couches (2, 3) de la partie structurelle, et où la structure de charpente présente des interruptions qui se situent dans différentes couches (2, 3) de la partie structurelle respectivement à des positions différentes, ne pas situées directement les unes au-dessus des autres, où les interruptions (5) sont disposées de telle sorte qu'à l'intérieur d'au moins une couche (2, 3) est formé un trajet de courant continu (6) s'étendant d'une zone d'extrémité de la partie structurelle à la zone d'extrémité opposée.

2. Implant ou instrument médical selon la revendication 1, **caractérisé en ce que** le trajet de courant continu (6) est réalisé en forme d'hélice.

3. Implant ou instrument médical selon la revendication 1, **caractérisé en ce que** les interruptions (5) sont disposées de telle sorte qu'à l'intérieur d'au moins deux couches superposées (2, 3) soient formées deux ou plus de sections de trajet de courant sensiblement en forme d'hélice (6', 6"), où les sections de trajet de courant (6', 6") de couches différentes (2, 3) de la partie structurelle sont disposées pour se recouvrir au moins partiellement.

4. Implant ou instrument médical selon l'une des revendications 1 à 3, **caractérisé en ce que** les trajets de courant (6) ou sections de trajet de courant (6', 6") continus formés à l'intérieur de différentes couches (2, 3) de la partie structurelle sont reliés les uns aux autres.

5. Implant ou instrument médical selon la revendication 4, **caractérisé en ce que** les trajets de courant (6) ou sections de trajet de courant (6', 6") sont reliés les uns aux autres par au moins une capacité électrique (8).

6. Implant ou instrument médical selon la revendication 5, **caractérisé en ce que** la capacité (8) est formée par des zones superposées, électriquement conductrices, des couches (2, 3) de la partie structurelle.

7. Implant ou instrument médical selon l'une des revendications 1 à 7, **caractérisé en ce que** les trajets de courant (6) ou sections de trajet de courant (6', 6") sont reliés entre eux par des métallisations des trous entre les couches (2, 3).

8. Implant ou instrument médical selon la revendication 2 ou 3, **caractérisé en ce que** les trajets de courant en forme d'hélice (6) ou sections de trajet de courant (6', 6") de couches différentes (2, 3) ont un sens de rotation opposé.

9. Implant ou instrument médical selon la revendication 5, **caractérisé en ce que** la capacité (8) et les inductances (7, 9) formées par les trajets de courant (6) ou sections de trajet de courant (6', 6") sont accordées de telle sorte qu'un résonnateur haute fréquence est formé dont la fréquence de résonance est égale à la fréquence de résonance d'un appareil RM.

10. Implant ou instrument médical selon l'une des revendications 1 à 9, **caractérisé en ce qu'**au moins deux des couches (2, 3) de la partie structurelle sont constituées de matériaux ayant des susceptibilités magnétiques opposées.

11. Implant ou instrument médical selon l'une des revendications 1 à 10, **caractérisé en ce que** les couches (2, 3) de la partie structurelle sont formées par deux ou plus d'éléments tubulaires disposés coaxialement.

12. Implant ou instrument médical selon l'une des revendications 1 à 11, **caractérisé en ce que** des couches (2, 3) réalisées en matériau électriquement conducteur de la partie structurelle sont séparées les unes des autres par des couches intermédiaires (4) en matériau électriquement isolant.
